# EUROPEAN PATENT SPECIFICATION

(11) **EP 1 678 499 B1**
(45) Date of publication and mention of the grant of the patent: **25.06.2008**
(21) Application number: 04793802.2
(22) Date of filing: 18.10.2004
(51) Int. Cl.: G01N 33/534, G01N 33/566, C07D 471/08, A61K 31/504, A61K 31/5025, A61P 9/06

(54) **CHEMICAL COMPOUND AND ASSAY**
CHEMISCHE VERBINDUNG UND TEST
COMPOSE CHIMIQUE ET DOSAGE

(30) Priority: 20.10.2003 SE 0302775
(43) Date of publication of application: 12.07.2006
(73) Proprietor: AstraZeneca AB, 151 85 Södertälje (SE)
(72) Inventor: SPRINGTHORPE, Brian, AstraZeneca R & D Charnwood, Loughborough, Leicestershire LE11 5RH (GB); STRANDLUND, Gert, S-431 83 Mölndal (SE)
(86) International application number: PCT/SE2004/001499
(87) International publication number: WO 2005/037052

(56) References cited:
- EP-A2- 0 308 843
- WO-A1-02/04446
- WO-A1-02/05860
- WO-A1-03/006988
- WO-A2-03/021271
- DE-A1- 2 428 792
- DE-A1- 3 730 224
- US-A- 4 550 112
- MANOTTI LANFREDI A.M. ET AL: 'Iridium(III) cis-Dihydrido complexes with 3,6-Bis(2'-pyridyl)pyrizidane. Chrystal Structure of the Pyridazinyl Complex ((IrH2(PPh3)2(u-C4HN2(C5H4N)2-3,6))PF6' J CHEM SOC DALTON TRANS no. 1, 1988, pages 651 - 656, XP002986104
- CHIAVARELLI S. ET AL: 'Sintesi nella serie dell' 1,5-difenilbispidin-9-ONE' IL FARMACO vol. 20, no. 6, 1965, pages 108 - 420, XP002986105

## Description

### TECHNICAL FIELD

The present invention relates to a labelled ligand compound that is useful in a method of identifying, testing and/or screening of compounds modulating ion channels, in particular myocardial I_{Kr} channels such as those encoded by ERG, including hERG. The ligand compound is therefore of use to evaluate the affinity of preclinical compounds at the ERG potassium channel.

### BACKGROUND TO INVENTION

It is now recognised that some drug-induced sudden deaths are secondary to the development of an arrhythmia called Torsades de Pointes (TdP) (Vandenberg JI, Walker BD, Campbell TJ. HERG K+ channels: friend and foe. Trends Pharmacol Sci 2001; 22(5): 240-6).

Recent advances in the understanding of this phenomenon indicate that the primary event is inhibition of the rapid component of the delayed rectifying potassium current (I_{Kr}) by such drugs. These compounds bind to the pore-forming α sub-units of the channel protein carrying this current - sub-units that are encoded by the human ether-α-go-go-related gene (hERG). Since I_{Kr} plays a key role in repolarisation of the cardiac action potential, its inhibition slows repolarisation and this is manifested as a prolongation of the QT interval. Whilst QT interval prolongation is not a safety concern *per se,* it carries a high risk of cardiovascular adverse effects and in a small percentage of people it can lead to TdP and degeneration into ventricular fibrillation.

Many compounds fail to become marketable drugs because of inhibition of the myocardial I_{Kr} channel, encoded by hERG. Patients with LQT 2 syndrome, in which the human ether-a-go-go (hERG) gene is mutated also exhibit Torsades de Pointes. The hERG channel contains a binding site, for example, for the class III antiarrhythmic methanesulphonanilides (dofetilide, E-4031 and MK-449) and many drugs that cause prolonged QT in the clinic share such a site. Consequently they have either warning labels (e.g. pimozide) or have been withdrawn from the market (e.g. terfenadine). The lack of an appropriate therapeutic margin between activity at the hERG channel and the desired target will prevent a potential drug from progressing further. It is therefore necessary to evaluate the hERG activity as early as possible to allow reduction in this activity for novel compound classes.

In order to evaluate ERG, and, in particular, hERG, activity and binding of novel compounds a suitable assay is needed. Preclinical Assays have been developed using radiolabelled compounds which bind to hERG and allow displacement studies to determine the affinity of novel compound classes. However, there is a need for alternative ligands for use in such assays.

### STATEMENT OF INVENTION

WO 02/04446 discloses bispidine compounds and their use in the treatment of cardiac arrhythmias.

Accordingly, in first aspect, the present invention relates to a radiolabelled derivative of a bispidine compound as described in WO 02/04446 or salts, hydrates or solvates thereof. Suitably said compound comprises radiolabelled substitutions and, in particular, tritium substitutions. In a preferred embodiment, the compound comprises at least 1, 2 or 3 tritium substitutions.

In a preferred embodiment, the invention relates to a compound having Formula I or salts, hydrates or solvates thereof and comprising at least one radiolabel:

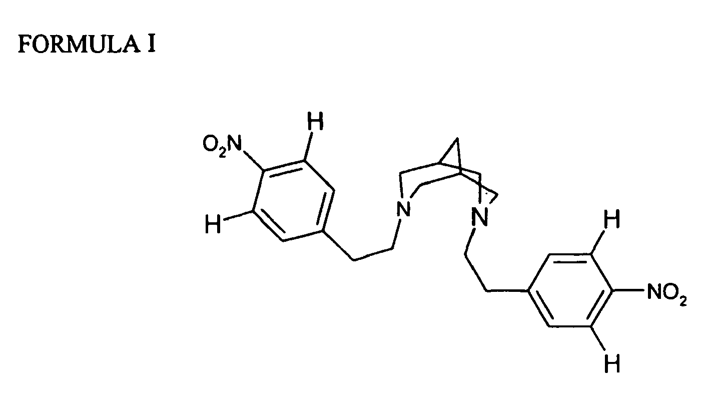

Suitably said compound comprises at least 1, 2 or 3 tritium substitutions preferably ortho to the nitro group.

Such radioligands or radiolabelled compounds are useful to evaluate affinity of compounds at the I_{Kr} channel encoded by ERG, and in particular hERG.

In a preferred embodiment of the invention there is provided a compound of Formula II which is: 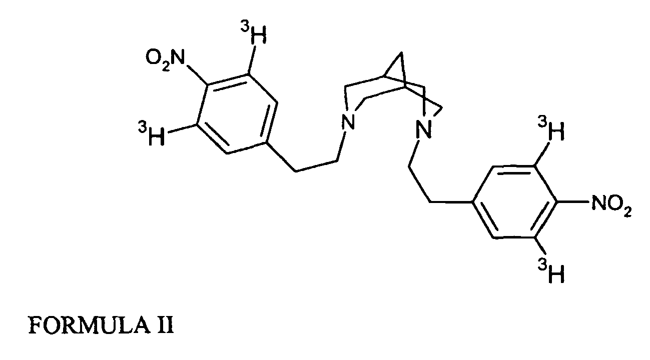 or salts thereof.

Suitable salts include acid addition or base salts thereof. A review of some suitable salts may be found in Berge et al, J Pharm Sci, 66, 1-19 (1977). Salts are formed, for example with strong inorganic acids such as mineral acids, e.g. sulfuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or terephthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid.

The invention also includes all enantiomers and tautomers of the radiolabelled compound of Formula I or II. The person skilled in the art will recognise compounds that possess optical properties (one or more chiral carbon atoms) or tautomeric characteristics. The corresponding enantiomers and/or tautomers may be isolated/prepared by methods known in the art.

In addition, the invention includes any stereoisomers and/or geometric isomers of the radiolabelled compound of Formula I or II. For example there may be one or more asymmetric and/or geometric centres and so the compound may exist in two or more stereoisomeric and/or geometric forms. The present invention contemplates the use of all the individual stereoisomers and geometric isomers of those agents, and mixtures thereof. The terms used in the claims encompass these forms, provided said forms retain the appropriate functional activity (though not necessarily to the same degree).

The present invention also includes other isotopic variations of the compound or its salt. An isotopic variation of an agent of the present invention or a pharmaceutically acceptable salt thereof is defined as one in which at least one atom is replaced by an atom having the same atomic number but an atomic mass different from the atomic mass most commonly found in nature. Examples of further isotopes that can be incorporated into the compound include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. As set out in Formula II, the tritiated form, i.e., ³H is particularly preferred. Carbon-14, i.e., ¹⁴C, isotopes may also be used and are preferred for their ease of preparation and detectability. Isotopic variations can generally be prepared by conventional procedures using appropriate isotopic variations of suitable reagents.

The present invention also includes the use of solvate forms of the compound. The terms used in the claims encompass these forms.

The successful use of a radioligand compound in a binding assay relies on a number of parameters including the affinity of the radioligand for the channel of interest as well as the off rate of the compound once it has bound. Ideally a suitable radioligand will have an affinity that allows competition with a candidate compound which can bind the same site as well as an off rate that allows the radioligand to remain in contact long enough for its binding to be detectable. These properties in a compound cannot be predicted. Accordingly, the present invention relates to the finding that a 1, 2 or 3 tritium substituted compound of Formula I or a compound of Formula II can be synthesised and is suitable for use in a competitive binding assay.

In a second aspect of the invention there is provided a method for characterising the activity of a compound as an I_{Kr} channel blocker involving using a compound in accordance with the invention.

Suitably said method is an assay comprising the following steps:
a) incubation of cell membrane containing the I_{Kr} channel in the presence of the radioligand compound of Formula II in the presence or absence of a test compound or a mixture of test compounds;
b) quantitation of specifically bound labelled compound in the presence or absence of a test compound;
c) calculation of the inhibition of labelled compound binding by the test compound or mixture of test compounds.

Such an assay is useful for the identification and characterisation of compounds that have potentially cardiotoxic side effects. The assay measures the ability of the test compound to displace the radioligand compound of Formula II from the Iₖᵣ channel which is, preferably, the ERG channel. Suitably the assay can be performed in a high throughput test system. The assay enables suitable compounds (i.e. those having no, low or reduced affinity to the Iₖᵣ channel) to be selected for further development..

Alternatively, such an assay is useful in identifying compounds that are capable of binding the Iₖᵣ channel and therefore potentially useful in the treatment of patients with arrythmias.

In a preferred embodiment, said assay comprises the steps of :
a) preparing solutions of test compound at one or more concentrations;
b) mixing the radioligand compound of Formula II with the cell membrane containing the I_{Kr} channel;
c) incubating the solutions of test compound with the mixture of radioligand compound of Formula II and cell membrane containing the I_{Kr} channel;
d) isolating the membrane from the solutions and measuring the radioactivity of the membrane;
e) calculating the radioactivity of samples in the presence of test compound compared to a control in the absence of test compound.

Suitably, the concentration of the test compound that gives 50% inhibition of binding of the radioligand compound to the cell membrane (IC₅₀) is calculated. These values can be used to predict the concentration of the compound liable to cause undesirable side effects in humans.

In one embodiment, the assay is a spot test. In another embodiment, the assay tests a range of concentrations and, preferably, five or more concentrations.

Suitable methods for isolating the membrane and measuring radioactivity include filter binding assays such as the assay described herein. Alternatively, radioactivity may be measured using a bead based assay such as a scintillation proximity assay (SPA, Amersham Biosciences).

Preferably the I_{Kr} channel is human ERG (hERG/HERG).

In one embodiment the cell membrane comprising the Iₖᵣ channel is derived from a cell line transfected with the ERG gene. Suitably, the ERG gene is human (hERG), primate or canine.

HERG has been expressed as stable or transiently expressed functional channels in human embryonic kidney (HEK) cells (see, for example, Circulation 2001 Nov 27;104(22):2645-8 Phospholipid metabolite 1-palmitoyl-lysophosphatidylcholine enhances human ether-a-go-go-related gene (HERG) K(+) channel function. Wang J, Wang H, Han H, Zhang Y, Yang B, Nattel S, Wang Z; J Biol Chem 1998 Aug 14;273(33):21061-6 HERG channel dysfunction in human long QT syndrome. Intracellular transport and functional defects. Zhou Z, Gong Q, Epstein ML, January CT).

Accordingly, suitable cell lines include HEK (human embryonic kidney) cells such as HEK 293 cells. Other suitable cell lines include CHO (chinese hamster ovary), CHL (chinese hamster lung) or COS (monkey) cells. In addition, the channel may be expressed in bacterial, yeast or insect cells such as SF9.

In another aspect of the invention, there is provided a use of a compound of the invention in an assay for characterising the I_{Kr} channel blocker activity of one or more test compounds.

In a further aspect, there is provided a use of a compound of Formula II in an assay for identifying one or more candidate compounds capable of binding to an Iₖᵣchannel.

Suitably, the assay is a competitive binding assay.

In yet another aspect of the invention there is provided a process for preparing a compound of Formula II as defined herein, said process comprising tritiating 3,7-Bis[2-(4-nitrophenyl)ethyl]-3,7-diazabicyclo[3.3.1]nonane in the presence of (1,5-cyclooctadiene)bis(methyldiphenyl-phosphine)iridium(I) hexafluorophosphate.

Suitably, the 3,7-Bis[2-(4-nitrophenyl)ethyl]-3,7-diazabicyclo[3.3.1]nonane and (1,5-cyclooctadiene)bis(methyldiphenyl-phosphine)iridium(I) hexafluorophosphate are dissolved in dichloromethane.

In one embodiment, tritiation is carried out using a tritiation manifold.

In a particularly preferred embodiment, the process for preparing a compound of Formula II is substantially as described herein.

The following non-limiting examples show the use of the synthesis and use of the compound with Formula II.

### EXAMPLES

### EXAMPLE 1- SYNTHESIS OF RADIOACTIVE LIGAND

**SYNTHESIS OF 3,7-BIS[2-(4-NITROPHENYL)ETHYL]-3,7-DIAZABICYCLO[3.3.1]NONANE PRIOR TO RADIOLABELLING IS DESCRIBED IN** WO 02/04446**.**

Radiolabelling to obtain the compound of Formula II: 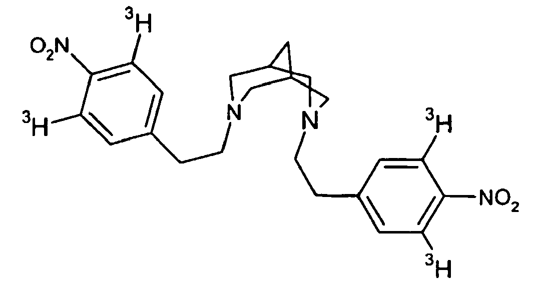 3,7-Bis[2-(4-nitro[3,5-³H]phenyl)ethyl]-3,7-diazabicyclo[3.3.11]nonane (FORMULA II)
is carried out as follows:
3,7-Bis[2-(4-nitrophenyl)ethyl]-3,7-diazabicyclo[3.3.1]nonane (1.62mg, 3.82µmol) and (1,5-cyclooctadiene)bis(methyldiphenyl-phosphine)iridium(I) hexafluorophosphate (8.6mg, 10.3µmol) are dissolved in dichloromethane (1ml) and transferred to a 2ml heavy glass-walled round bottomed flask. The flask is attached to an RC Tritec tritiation manifold and subjected to a cycle of freezing, evacuating to <0.01 mbar and thawing to degas the sample. The dichloromethane solution is finally frozen in liquid nitrogen and tritium gas (approximately 130GBq, 61 µmol), generated from the primary uranium bed on the manifold, is introduced into the void above the solution. The reaction is allowed to warm to room temperature and stirred for 20 hours, via means of a magnetic stirrer bar.

The solution is refrozen in liquid nitrogen and residual tritium gas reabsorbed onto the secondary uranium storage bed of the manifold. The flask is removed from the manifold and the solution subjected to two cycles of lyophilisation using ethanol (2 x 5ml) to remove labile tritium. The resulting residue is dissolved in ethanol (10ml) to afford the stock solution of 3,7-Bis[2-(4-nitro[3,5-³H]phenyl)ethyl]-3,7-diazabicyclo[3.3.1]nonane (19.33 GBq, radiochemical purity 34%).

An aliquot (2ml, 3.87GBq) of the stock solution is taken and blown down under a stream of nitrogen and the residue redissolved in acetonitrile/0.5% v/v aqueous trifluoroacetic acid, 1:1 by volume (400µl). The sample is purified in approximately five equal injections using the following HPLC conditions: Xterra MS C-8 150 x 8 mm, 35% acetonitrile/0.5% v/v aqueous trifluoroacetic acid at 3 ml min⁻¹, detection 240 nm. The fraction due to 3,7-Bis[2-(4-nitrophenyl)ethyl]-3,7-diazabicyclo[3.3.1]nonane (retention time 10.77min) is collected each time and combined (radiochemical purity >97%). Aqueous sodium thiosulphate (50% w/v, 50µl) is added to the combined fractions to stabilise the sample prior to removal of the organic solvent under reduced pressure. The resulting aqueous solution is freeze-dried to leave a white residue which is reconstituted in ethanol/water, 2:1 by volume (15ml) to afford the purified stock of 3,7-Bis[2-(4-nitro[3,5-³H]phenyl)ethyl]-3,7-diazabicyclo[3.3.1]nonane (881.7MBq; radiochemical purity 97.5%). The molar specific radioactivity is determined by mass spectrometry and is found to be 2941.5 GBq mmol⁻¹.

### Example 2 - Protocol for hERG Binding Assay

### Assay plates

96-well polypropylene plates (Costar C3600)
Whatman GF/B filter plates (Packard (6005177N))

### Assay and wash buffer

10mM HEPES
130mM NaCl
5mM KCl
1mM EGTA
0.8mM MgCl₂
pH 7.4 with NaOH

### GF/B plate coating solution

0.3% (v/v) Polyethylenimine
0.2% (w/v) BSA
GFB filter plates are pre-soaked in coating solution for a minimum of 20 minutes before harvesting.

### MEMBRANES

Membranes for use in the assay are prepared as follows:
HEK (human embryonic kidney) cells are grown using standard methods to give the required number, they are then harvested and pelleted. The final pellet is prepared in serum-free medium and the packed cell volume (pcv) measured.

The pellet is resuspended in 4 times the pcv in hypotonic buffer solution (3 parts water: 1 part serum free medium or buffer) with 1 tablet / 50ml of Boehringer protease inhibitor (Cat No 1 697 498) added.

The cells are allowed to swell for a couple of minutes on ice then lysed using a Polytron tissue homogeniser set to 22,000 rpm. The cell suspension is held on ice and the Polytron is allowed to build up to speed, then turned off and allowed to cool for 30 seconds before repeating. Normally 3 bursts should be sufficient to completely lyse the cells. A sample is checked microscopically to ensure complete lysis and the process repeated if necessary.

10ml of cold 41% sucrose solution in buffer is added to 38ml Beckman ultra centrifuge tube(s) (Cat No 344058). The lysed membrane solution is carefully overlaid over the 41% cushion. Any debris that may be stuck to the sides is washed out of the Polytron tube with buffer. The ultra centrifuge tube is topped up with cold buffer to completely fill the tube (this prevents the tube from collapsing during centrifugation). Centrifugation is carried out at 28,000 rpm for 1 hour @ 4°C using a SW-28 swing-out rotor with a brake to 800rpm.

Membranes are seen as a white band at sucrose / buffer interphase. The top layer of the buffer is discarded and then all the membrane band is harvested with as little sucrose as possible and added to a fresh centrifuge tube. This is diluted and mixed well with at least 3 volumes of ice cold buffer containing protease inhibitor. Centrifugation is carried out at 23,000 rpm at 4°C for 20 minutes with a break to zero.

The supernatant is discarded and the pellet resuspended in the assay buffer + protease inhibitors on ice at 1 x 10⁸ cell equivalents/ml. This preparation is then aliquotted and frozen to -80°C or lower.

For use in the assay, the membrane solution is diluted 65-fold, e.g. 0.5 mL membranes diluted to 32.5 mL assay buffer per assay plate. Total binding of 3,7-Bis[2-(4-nitro[3,5-³H]phenyl)ethyl]-3,7-diazabicyclo[3.3.1]nonane does not exceed 10% of the total added.

### RADIOLABEL

3,7-Bis[2-(4-nitro[3,5-³H]phenyl)ethyl]-3,7-diazabicyclo[3.3.1]nonane is made according to the above protocol and supplied in 67% (v/v) ethanol, 33% (v/v) water containing 3.3 mg/mL sodium thiosulphate. The batch of radiolabel used in these studies has a specific activity of 2941.5 GBq/mmol (79.5 Ci/mmol) and a concentration of 20 µM. The stock radiolabel is diluted 2000-fold to 10 nM and 20 µL added per well to a final assay volume of 200 µL to give a final radioligand concentration of 1 nM.

### Total binding

Total binding, in the absence of a competing ligand, is defined in the presence of a vehicle control. In this case it was 1% (v/v) DMSO (20 µL per well from a stock of 10% (v/v) DMSO in assay buffer).

### NSB

Non-specific binding is determined by measuring the binding of 3,7-Bis[2-(4-nitro[3,5-³H]phenyl)ethyl]-3,7-diazabicyclo[3.3.1]nonane in the presence of 10 µM astemizole (20 µL per well from a stock of 100 µM in 10% (v/v) DMSO in assay buffer).

### Test compounds

Test compounds are dissolved in DMSO to a stock concentration of 10 mM. Typically up to 7 compounds are tested per plate, with an internal standard. These are diluted simultaneously in a 96-well plate using an 8-channel pipette. Compounds are serially diluted in DMSO to 100x the final assay concentrations to be used, typically half log units from 3 mM to 30 µM. This is achieved by adding 30 µL of each successive dilution to 65 µL DMSO in a 96-well plate. Further dilutions are carried out in assay buffer as follows: compounds are diluted 10-fold with assay buffer to give 5 concentrations of compound in 10% DMSO. 20 µL of each of these dilutions is then added to the assay plate. When the other additions are made this makes a total volume of 200 µL and a final DMSO concentration of 1% (v/v).

### ASSAY STANDARD

Pimozide, a hERG-active compound, is used as the assay standard. Its pIC₅₀ was 7.7 ± 0.1 (mean ± SEM, n=7, range 7.5 - 8.0). The compound is made up as 10 mM stock in DMSO and frozen in aliquots at -20°C for use in each experiment. One standard compound is tested in each assay plate.

### ASSAY PROCEDURE

Assays are performed in Costar polypropylene round-bottomed 96 well plates. Each assay plate contains controls for total binding and non-specific binding. Compounds are usually tested at five log step dilutions, in duplicate. This allows eight compounds to be tested per assay plate. Typically compounds are tested over the range 30 µM to 0.3 µM. Additions to each assay plate are summarised in Table 1:

**Table 1 Well contents for assay plates**

| | Total Binding | Binding + Compound | Non-Specific Binding |
|---|---|---|---|
| Compound | -- | 20 µL | -- |
| Vehicle | 20 µL | -- | -- |
| Compound of | 20 µL | 20 µL | 20 µL |
| Formula II | | | |
| 10 µM astemizole | -- | -- | 20 µL |
| Membranes | 160 µL | 160 µL | 160 µL |

After all the additions are made the plates are placed on a plate shaker for 1 minute to allow mixing. The plates are then incubated for 3 hours at room temperature (18-20°C). During this time one GF/B plate for each assay plate is immersed in coating solution. After incubation the assay well contents are harvested using a Tomtec harvester and washed seven times with approximately 250 µL wash buffer per cycle (1750 µL total). The plate layout is transposed in the harvester so that well A1 in the assay plate is harvested onto well A12 in the GF/B plate.

20 µL of 10 nM 3,7-Bis[2-(4-nitro[3,5-³H]phenyl)ethyl]-3,7-diazabicyclo[3.3.1]nonane is added to a dry filter plate to determine the total radioactivity added to assay wells. The plates are dried either in an oven at 58°C for 2 hours or overnight at room temperature. A backseal is adhered to the underside of each plate and 50 µL/well Microscint 0 is added onto each filter. A TopSeal is added to each plate and the radioactivity counted with a Packard TopCount on a [³H] protocol with the inverted plate orientation enabled to correct for the transposed layout performed during filtering.

### Data analysis

The mean CPM values for total binding, non-specific binding and total [³H] added are calculated. The mean CPM value is also calculated for each concentration of test compound and the mean NSB subtracted. A concentration-effect curve for each test compound is constructed by plotting the mean CPM against the concentration of test compound. The pIC₅₀ value is then determined.

### RESULTS

3,7-Bis[2-(4-nitro[3,5-³H]phenyl)ethyl]-3,7-diazabicyclo[3.3.1]nonane bound reversibly to membranes from hERG-transfected HEK cells with high affinity (Kd 0.91 ± 0.01 nM; Bmax 23.1 ± 0.4 pmol/mg protein; mean ± SEM, n=3).

For competition displacement assays a radioligand concentration of 1nM is used which provides a binding window of 10-fold over non-specific binding and demonstrated reversible, single site binding characteristics.

### Comparison with published [³H]-dofetilide data

3,7-Bis[2-(4-nitro[3,5-³H]phenyl)ethyl]-3,7-diazabicyclo[3.3.1]nonane is fully displaced from hERG-transfected HEK membranes by a range of compounds previously described as hERG channel blockers.

**Table 2. Comparison of pIC₅₀ values for known hERG channel blockers derived from competition binding assays using 3,7-Bis[2-(4-nitro[3,5-³H]phenyl)ethyl]-3,7-diazabicyclo[3.3.1]nonane and [³H]-dofetilide (Finlayson et al.) in hERG-transfected HEK membranes (mean ± SEM, n ≥4).**

| Ligand | 3,7-Bis[2-(4-nitro[3,5-³H]phenyl)ethyl]-3,7-diazabicyclo[3.3.1]nonane pIC₅₀ | [³H]-Dofetlide pIC₅₀ |
|---|---|---|
| 3,7-Bis[2-(4-nitrophenyl)ethyl]-3,7-diazabicyclo[3.3.1]nonane | 8.5 | - |
| Astemizole | 7.9 | - |
| Dofetilide | 7.4 | 7.2 |
| Cisapride | 6.5 | - |
| Terfenadine | 6.4 | 6.4 |
| Pimozide | 7.7 | 7.2 |
| E-4031 | 6.9 | 6.6 |
| Clofilium | 8.7 | 8.1 |
| d-Sotalol | 4.6 | 3.9 |
| Haloperidol | 6.1 | 6.3 |

The pIC₅₀ values for these compounds agree well with published data observed using Pfizer's dofetilide as the radioligand (Table 2; data from Finlayson *et al*.). This correlation is consistent with the view that 3,7-Bis[2-(4-nitro[3,5-³H]phenyl)ethyl]-3,7-diazabicyclo[3.3.1]nonane occupies a similar binding site to that occupied by many of the drugs known to interact with the hERG channel.

### Comparison with electrophysiology data for known hERG blockers

A comparison of the 3,7-Bis[2-(4-nitro[3,5-³H]phenyl)ethyl]-3,7-diazabicyclo[3.3.1]nonane binding pIC₅₀ values with pIC₅₀ values from electrophysiology for these hERG channel blockers shows that the binding assay can predict those compounds that will interact functionally with the channel (Table 3).

**Table 3. Comparison of pIC₅₀ values for known hERG channel blockers derived from competition binding assays using 3,7-Bis[2-(4-nitro[3,5-³H]phenyl)ethyl]-3,7-diazabicyclo[3.3.1]nonane and electrophysiology.**

| Ligand | 3,7-Bis[2-(4-nitro[3,5-³H]phenyl)ethyl]-3,7-diazabicyclo[3.3.1]nonane pIC₅₀ | Electrophysiolo gy pIC₅₀ | Source of data Human unless Human unless stated |
|---|---|---|---|
| Dofetilide | 7.4 | 6.8 | In-house |
| Cisapride | 6.5 | 7.1 | In-house |
| Terfenadine | 6.4 | 7.3 | In-house |
| Pimozide | 7.7 | 7.7 | Kang 2000 |
| E4031 | 6.9 | 7.1 | In-house |
| d-Sotalol | 4.6 | 4.1 | Chadwick 1993;G.Pig |
| Haloperidol | 6.1 | 6.0 | Suessbrich 1997 |

### Comparison with electrophysiology data for a range of candidate compounds

A range of compounds representing a diverse range of structural classes are screened in the 3,7-Bis[2-(4-nitro[3,5-³H]phenyl)ethyl]-3,7-diazabicyclo[3.3.1]nonane binding assay. Comparison of these data with functional pIC₅₀ data from the electrophysiology studies shows that the majority of compounds were 3 to 10-fold less active in the binding assay than in electrophysiology. However, all compounds with functional activity of greater than 10 micromolar were detected in the binding assay.

The data for the standard compounds indicates that the binding assay would be appropriate for investigating SAR of channel blockers. In contrast to the standards, when dealing with much less potent compounds as is usual for project compounds, clear SAR is not within the sensitivity of the assay.

What the binding assay clearly provides is a convenient means of rapid, early detection of the ability of project compounds to interact with the hERG channel.

### Conclusion

A simple, robust radioligand-binding assay has been developed, which identifies hERG-binding activity in a diverse range of structural classes.

For the majority of compounds the activity in the binding assay was lower than the activity determined by electrophysiology but the overall positive correlation between the screens ensures that compound classes with significant activity in the functional screen will be detected in the binding assay. The binding assay can be used to detect compound classes with potential to cause QT prolongation.

This binding assay is suitable for use as a primary hERG screen, minimising the number of compounds that need to be tested by electrophysiology.

### REFERENCES

1) Chadwick C., et al., Identification of a specific ligand for the cardiac rapidly activating delayed rectifier K+ current. Circ. Res. (1993) 72:707-714.
2) Finlayson K., et al., [3H]dofetilide binding to hERG-transfected membranes: a potential high throughput preclinical screen. Eur. J. Pharmacol. (2001) 430:147-148.
3) Kang J., et al., High affinity blockade of the hERG cardiac K+ channel by the neuroleptic pimozide. Eur. J. Pharmacol. (2000) 392:137-140.
4) Suessbrich H., et al., The inhibitory effect of the antipsychotic drug haliperidol on hERG potassium channels expressed in Xenopus oocytes. British Journal of Pharmacol. (1997) 120:968-974.

Although the invention has been described in connection with specific preferred embodiments, it should be understood that the invention as claimed should not be unduly limited to such specific embodiments. Insteed, various modifications of the described modes for carrying out the invention which are obvious to those skilled in chemistry or related fields are intended to be within the scope of the following claims.

## Claims

1. A compound having Formula I or salts, hydrates or solvates thereof and comprising at least one radiolabel: 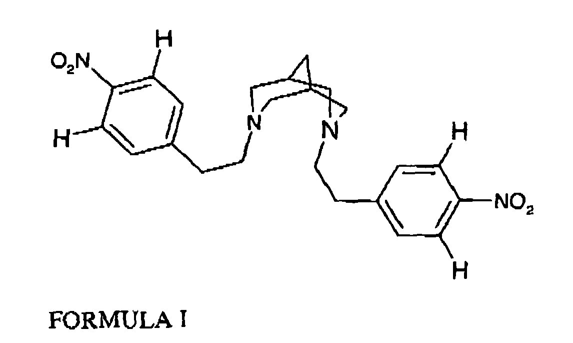 wherein the compound comprises at least 1, 2 or 3 tritium substitutions in the meta position.

2. A compound of Formula II: 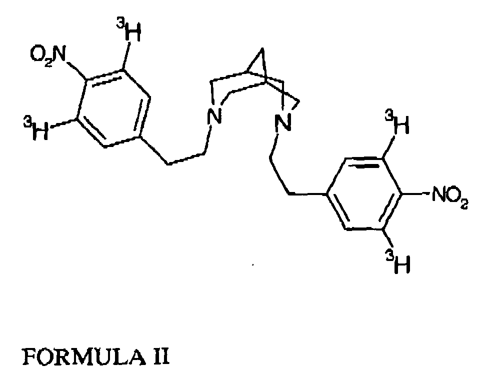 or salts thereof.

3. An assay for characterising the activity of a compound as an I_{Kr} channel blocker comprising the following steps:
a) incubation of cell membrane containing the I_{Kr} channel in the presence of the compound of Formula II in the presence or absence of a test compound;
b) determination of specifically bound labelled compound in the presence or absence of a test compound;
c) calculation of the inhibition of labelled compound binding by the test compound.

4. An assay as claimed in claim 3 comprising the steps of :
a) preparing solutions of test compound at one or more concentrations;
b) mixing the compound of Formula II with the cell membrane containing the I_{Kr} channel:
c) incubating the solutions of test compound with the mixture of compound of Formula 11 and cell membrane containing the I_{Kr} channel;
d) isolating the membrane from the solutions and measuring the radioactivity of the membrane;
e) calculating the radioactivity of samples in the presence of test compound compared to a control in the absence of test compound.

5. An assay as claimed in claim 3 or claim 4 wherein the I_{Kr} channel is human ERG.

6. An assay as claimed in claim 5 wherein the cell membrane is derived from a cell line transfected with the human ERG gene.

7. An assay as claimed in claim 6 wherein the cell line is HEK.

8. Use of a compound of Formula II in an in vitro assay for characterising the Iₖᵣ channel blocker activity of one or more candidate compounds.

9. A use as claimed in claim 8 wherein the assay is a competitive binding assay.

10. A process for preparing a compound of Formula II as defined in claim 2, said process comprising tritiating 3,7-Bis[2-(4-nitrophenyl)ethyl]-3,7-diazabicyclo[3.3.1]nonane in the presence of (1,5-cyclooctadiene)bis(methyldiphenyl-phosphine)iridium(I) hexafluorophosphate.

11. A process as claimed in claim 10 wherein the 3,7-Bis[2-(4-nitrophenyl)ethyl]-3.7-diazabicyclo[3.3.1]nonane and (1,5-cyclooctadiene)bis(methyldiphenylphosphine)iridium(I) hexafluorophosphate are dissolved in dichloromethane.

12. A process as claimed in claim 10 or claim 11 wherein tritiation is carried out using a tritiation manifold.

## Patentansprüche

1. Verbindung der Formel I und deren Salze, Hydrate und Solvate, die wenigstens einen radioaktiven Marker enthalten: 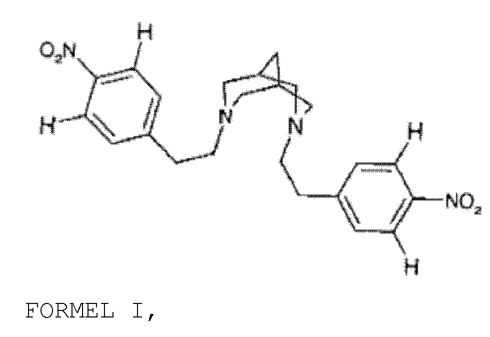 wobei die Verbindungen wenigstens 1, 2 oder 3 Tritiumsubstitutionen in der meta-Stellung enthalten.

2. Verbindung der Formel II 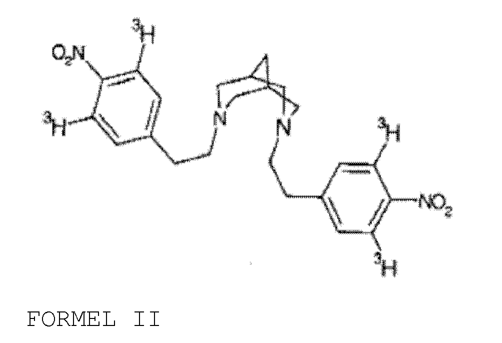 und deren Salze.

3. Assay zur Charakterisierung der Aktivität einer Verbindung als I_{Kr}-Kanalblocker, welcher die folgenden Schritte umfaßt:
a) Inkubieren der den I_{Kr}-Kanal enthaltenden Zellmembran in Gegenwart einer Verbindung der Formel II in Gegenwart oder Abwesenheit einer Testverbindung;
b) Bestimmung von spezifisch gebundener markierter Verbindung in Gegenwart oder Abwesenheit einer Testverbindung;
c) Berechnung der Inhibierung der Bindung der markierten Verbindung durch die Testverbindung.

4. Assay nach Anspruch 3, welcher die folgenden Schritte umfaßt:
a) Herstellen von Lösungen der Testverbindung in einer oder mehreren Konzentrationen;
b) Mischen der Verbindung der Formel II mit der den I_{Kr}-Kanal enthaltenden Zellmembran;
c) Inkubieren der Lösungen der Testverbindung mit der Mischung der Verbindung der Formel II und der den I_{Kr}-Kanal enthaltenden Zellmembran;
d) Isolieren der Membran aus den Lösungen und Messen der Radioaktivität der Membran;
e) Berechnen der Radioaktivität der Proben in Gegenwart von Testverbindung verglichen mit einer Kontrolle in Abwesenheit von Testverbindung.

5. Assay nach Anspruch 3 oder 4, wobei es sich bei dem I_{Kr}-Kanal um humanes ERG handelt.

6. Assay nach Anspruch 5, wobei die Zellmembran aus einer mit dem humanen ERG-Gen transfizierten Zellinie gewonnen wurde.

7. Assay nach Anspruch 6, wobei es sich bei der Zellinie um HEK handelt.

8. Verwendung einer Verbindung der Formel II in einem in-vitro-Assay zur Charakterisierung der I_{Kr}-Kanalblockeraktivität einer oder mehrerer Verbindungskandidaten.

9. Verwendung nach Anspruch 8, wobei es sich bei dem Assay um einen kompetitiven Bindungsassay handelt.

10. Verfahren zur Herstellung einer Verbindung der Formel II nach Anspruch 2, bei dem man 3,7-Bis[2-(4-nitrophenyl)ethyl]-3,7-diazabicyclo[3.3.1]nonan in Gegenwart von (1,5-Cyclooctadien)bis(methyldiphenylphosphin)iridium(I)-hexafluorphosphat tritiiert.

11. Verfahren nach Anspruch 10, wobei das 3,7-Bis[2-(4-nitrophenyl)ethyl]-3,7-diazabicyclo[3.3.1]nonan und das (1, 5-Cyclooctadien)bis(methyldiphenylphosphin)iridium(I)-hexafluorphosphat in Dichlormethan gelöst werden.

12. Verfahren nach Anspruch 10 oder 11, wobei die Tritiierung unter Verwendung eines Tritiierungsverteilers durchgeführt wird.

## Revendications

1. Composé de formule I ou des sels, des hydrates ou des solvates de celui-ci et comprenant au moins un radiomarquage : 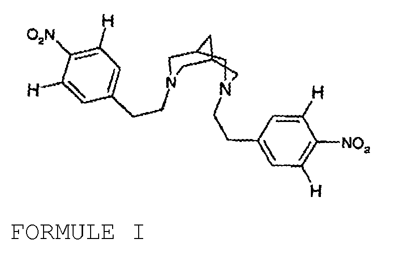 le composé comprenant au moins 1, 2 ou 3 substitutions par le tritium en position méta.

2. Composé de formule II : 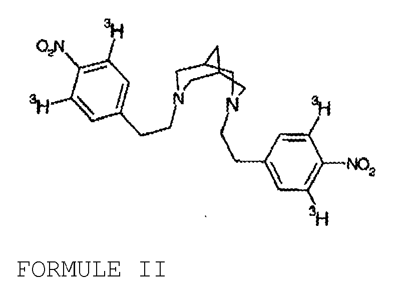 ou des sels de celui-ci.

3. Dosage pour caractériser l'activité d'un composé en tant que bloqueur de canal I_{Kr} comprenant les étapes suivantes :
a) l'incubation de membrane cellulaire contenant le canal I_{Kr} en présence du composé de formule II en présence ou en l'absence d'un composé à tester ;
b) la détermination du composé marqué lié de manière spécifique en présence ou en l'absence d'un composé à tester ;
c) le calcul de l'inhibition de la liaison de composé marqué par le composé à tester.

4. Dosage selon la revendication 3, comprenant les étapes consistant à :
a) préparer des solutions de composé à tester à une ou plusieurs concentrations ;
b) mélanger le composé de formule II avec la membrane cellulaire contenant le canal I_{Kr} ;
c) incuber les solutions du composé à tester avec le mélange de composé de formule II et la membrane cellulaire contenant le canal I_{Kr} ;
d) isoler la membrane des solutions et mesurer la radioactivité de la membrane ;
e) calculer la radioactivité des échantillons en présence du composé à tester par rapport à un témoin en l'absence du composé à tester.

5. Dosage selon la revendication 3 ou la revendication 4, **caractérisé en ce que** le canal I_{Kr} est l'EGR humain.

6. Dosage selon la revendication 5, **caractérisé en ce que** la membrane cellulaire est issue d'une lignée cellulaire transfectée avec le gène EGR humain.

7. Dosage selon la revendication 6, **caractérisé en ce que** la lignée cellulaire est HEK.

8. Utilisation d'un composé de formule II dans un dosage in vitro pour caractériser l'activité de bloqueur de canal I_{Kr} d'un ou de plusieurs composés candidats.

9. Utilisation selon la revendication 8, **caractérisée en ce que** le dosage est un dosage de liaison compétitive.

10. Procédé de préparation d'un composé de formule II tel que défini dans la revendication 2, ledit procédé comprenant la tritiation de 3,7-bis[2-(4-nitrophényl)éthyl]-3,7-diazabicyclo[3,3,1]nonane en présence d'hexafluorophosphate de (1,5-cyclooctadiène)bis(méthyldiphényl-phosphine)iridium(I).

11. Procédé selon la revendication 10, **caractérisé en ce que** le 3,7-bis[2-(4-nitrophényl)éthyl]-3,7-diazabicyclo[3,3,1]nonane et l'hexafluorophosphate de (1,5-cyclooctadiène)bis(méthyldiphénylphosphine)iridium(I) sont dissous dans du dichlorométhane.

12. Procédé selon la revendication 10 ou la revendication 11, **caractérisé en ce que** la tritiation est effectuée en utilisant une rampe de tritiation.
